# EUROPEAN PATENT SPECIFICATION

(11) **EP 1 423 199 B1**
(45) Date of publication and mention of the grant of the patent: **28.11.2007**
(21) Application number: 02755320.5
(22) Date of filing: 04.09.2002
(51) Int. Cl.: B01L 3/00, G01N 33/48

(54) **DEVICE FOR USE IN FLUID ASSAY**
VORRICHTUNG ZUR ANWENDUNG BEI DER UNTERSUCHUNG EINER BIOLOGISCHEN FLÜSSIGKEIT
DISPOSITIF PERMETTANT L'ANALYSE DE FLUIDES

(30) Priority: 04.09.2001 GB 0121340
(43) Date of publication of application: 02.06.2004
(73) Proprietor: PB Diagnostics Limited, LONDON EC3A 7EE (GB)
(72) Inventor: PERCIVAL, David Alan, Hawarden, Flintshire CH5 3HS (GB); PLUMPTRE, David Aubrey, Droitwich, Worcestershire WR9 7DJ (GB)
(74) Representative: McCall, John Douglas
(86) International application number: PCT/GB2002/004039
(87) International publication number: WO 2003/020424

(56) References cited:
- WO-A-93/03842
- US-A- 4 761 381
- US-A- 4 906 439

## Description

The present invention relates to a device, apparatus and method for use in fluid assay. In particular it relates to a device for use in body fluid assay, for example assays on blood and urine. More particularly, it relates to a device for use in assays for glycated proteins in body fluid samples.

PCT/GB98/03586 discloses an apparatus for conducting an assay comprising a first inlet, a second inlet, and an inlet port, the inlet port being moveable relative to each of the said first and second inlets such that the port can be brought into liquid communication with each inlet in turn as required, the inlet port accommodating a filter means and/or a binder retaining means. In the course of conducting an assay, for example to determine the presence or absence of one or more analytes in a sample, the sample is separated into a first component fraction and a second component fraction, the second component fraction being obtained by eluting a compound held on the binder retaining means.

United Kingdom Patent Application No: 0021887.5 discloses a device for conducting a fluid assay which comprises a carousel mounted on a hub which has an assay component separation zone. The carousel is rotatable about the hub which provides multiple configurations of the device. The carousel has a plurality of chambers which are non-communicating in a first configuration of the device. The device further comprises a first chamber which communicates through the separation zone with at least one other chamber in a second configuration of the device.

United States Patent No: 5935858 relates to a device and method for the isolation of one or more components of a sample. The disclosed device includes a reaction chamber, at least one reagent chamber containing a sealed, predetermined amount of reagent, at least one waste chamber, an entry port and a mechanism for moving the reaction chamber sequentially into fluid communication with each of these other components.

United States Patent No: 5573951 discloses a multi-chambered blood culture device for simultaneously conducting two blood culture tests on a single blood sample. This disclosure provides an integrated unit with a rotating blood inlet valve assembly for receiving a blood sample simultaneously into two independent sample vials. Independent culture chambers contain growth media, one culture chamber preferably containing an aerobic growth medium and another culture chamber preferably containing an anaerobic growth medium. The device is configured to isolate the sample vials and culture chambers from contaminants of the external environment. By rotating the blood inlet valve assembly, blood is simultaneously released from one sample vile into one culture chamber and from the other sample vial into the other culture chamber.

It is an object of the present invention to provide a device, apparatus and method for use in fluid assay which facilitates measurement of a predetermined quantity of fluid sample to be assayed.

The present invention accordingly provides a device for use in fluid assay comprising a sample reservoir for a fluid sample to be assayed, the reservoir having an inlet port and an outlet port, the device comprising a fluid sample receiving zone in fluid communication with the reservoir via the inlet port and an excess fluid sample detection zone in fluid communication with the reservoir via the outlet port, wherein the device has a first configuration in which fluid communication between the reservoir and each of the fluid sample receiving zone and the excess fluid sample detection zone is maintained and a second configuration in which one or each of said fluid communications is broken; The measured quantity of fluid sample present in the reservoir can be detached from any fluid sample present in the fluid sample receiving zone, the excess fluid sample detection zone or the communicating regions therebetween (including any reservoir overflow region) and then supplied to a fluid assay means, for example the assay device disclosed in United Kingdom Patent Application No: 0021887.5.

The device according to the invention enables a user to dispense accurately and conveniently a predetermined quantity of a fluid sample to be assayed into the reservoir. The known quantity of fluid sample supplied to the reservoir can then be assayed in accordance with known techniques.

In one preferred embodiment of the invention, fluid communication between the fluid sample receiving zone and the reservoir is at least partly achieved by capillary action. In this-embodiment of the device according to the invention, an unknown or approximate quantity of fluid sample is supplied to the fluid sample receiving zone and fluid sample is then transferred to the reservoir by means of capillary action, for example through a capillary tube.

In another preferred embodiment of the invention, fluid communication between the fluid sample detection zone and the reservoir is at least partly achieved by capillary action. Thus, as fluid sample is transferred to the reservoir and the reservoir fills up, fluid sample is drawn by capillary action, for example by means of a capillary tube, from the reservoir into the excess fluid sample detection zone.

Preferably, the reservoir has an overflow region and the excess fluid sample detection zone is in fluid communication with the overflow region of the reservoir.

In a further preferred embodiment of the invention, the excess fluid sample detection zone is viewable through a lens aperture. The lens aperture may be situated in the device. This provides a ready and convenient means for the operator to detect the presence of excess fluid sample in the excess fluid sample detection zone, the presence of such excess fluid sample indicating that the reservoir is full.

Preferably the reservoir of the device is sized to contain a preselected quantity of the sample to be assayed.

The device according to the invention may be co-operable with drive means for use in automated fluid assay.

The device according to the invention is intended for use in assaying fluid samples, particularly body fluid samples such as blood and urine.

Preferably, the device according to the invention is co-operable with means for assaying the fluid sample, for example means such as those disclosed in co-pending United Kingdom Patent Application No: 00218875

Accordingly, the invention further provides an apparatus for fluid assay comprising a device in accordance with the invention in co-operation with means for assaying the fluid sample.

The invention further provides a method for fluid assay comprising the steps of:
a) supplying a fluid to be assayed to the fluid sample receiving zone of a device in accordance with the invention;
b) detecting excess fluid sample to be assayed in the excess fluid sample detection zone of the device;
c) optionally supplying the fluid sample in the reservoir to a device adapted for assaying the sample; and
d) assaying the fluid sample.

In a particularly preferred embodiment of the invention, there is provided a device in accordance with the invention in co-operation with a further device for effecting fluid assay, the further device comprising a carousel mounted on a hub having an assay component separation zone, the carousel being rotatable about the hub to provide multiple configurations of the further device, the carousel having a plurality of chambers being non-communicating in a first configuration of the further device, a first chamber communicating through the separation zone with at least one other chamber in a second configuration of the further device. The further device is in accordance with co-pending U.K. Application No: 0021887.5. In this embodiment the hub of the further device may carry the device according to the invention. For example, the device of the invention may comprise a cap carried on the hub of the further device, the cap covering the carousel in use of the device, the cap comprising a reservoir having an inlet port and an outlet port and further comprising a fluid sample receiving zone in fluid communication with the reservoir via the inlet port and an excess fluid sample detection zone in fluid communication with the reservoir via the outlet port.

The device in accordance with the invention can be used in co-operation with a further device in accordance with co-pending UK Application No.0021887.5 further comprising a means for breaking an airtight seal disposed in at least one of the chambers.

A specific embodiment of the present invention will now be described with reference to the Figures by way of example only. The embodiment described is in accordance with the last of the preferred embodiments hereinbefore described with suitable adaptation the cap region of the described embodiment, which in itself is in accordance with the invention in its broader aspects, could be used in conjunction with the other types of fluid assay device.
Figure 1 is a perspective view of a device in accordance with the invention in co-operation with an assay component separation device according to U.K. 0021887.5. The assay component separation device is disassembled in Figure 1.
Figure 2 shows a perspective view of the device of Figure 1 after assembly of the assay component separation device but before receiving a blood sample to be assayed.
Figure 3 shows the device of Figure 2 after supply of the blood to be assayed.
Figure 4a shows a cut away perspective view of a device in accordance with the invention shortly after supply of body fluid thereto.
Figure 4b shows the device of Figure 4a a short time thereafter.
Figure 4c shows the device of Figure 4b a short while later.
Figure 4d shows the device of Figure 4c a short while later.

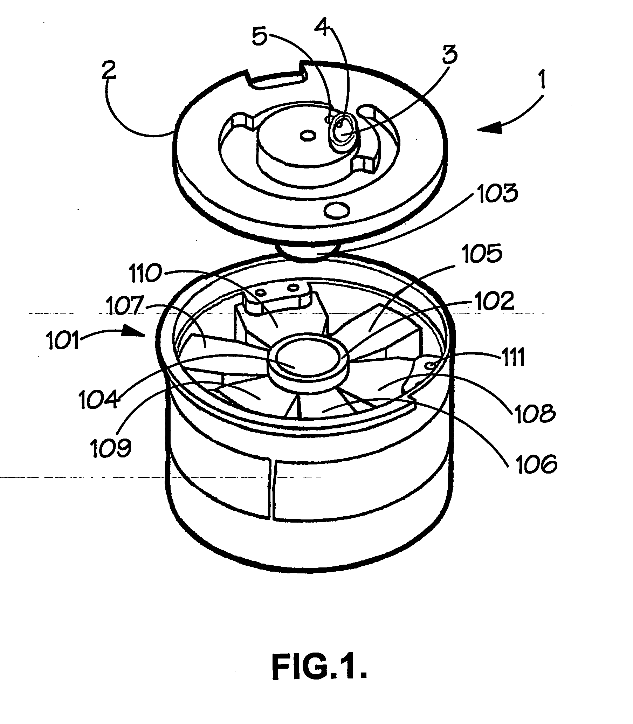

Referring to Figure 1 there is shown a device 1 in accordance with the invention in co-operation with an assay component separation device 101 according to co-pending United Kingdom Patent Application No: 0021887.5

Before describing device 1 in accordance with the invention, the working of assay component separation device 101 will now be briefly described.

Assay component separation device 101 comprises a carousel 102 rotatably mounted on a hub 103 which (when the assay component separation device is assembled, is disposed in a complimentary shaped housing 104. Carousel 102 is divided into a plurality of chambers. There are 3 optical chambers, 105, 106, 107 and 3 reagent chambers 108, 109, 110. The 6 chambers are radially disposed about hub 103 (when assembled) and are arranged into pairs which are disposed diametrically opposite one another. Each pair comprises a reagent chamber and an optical chamber. In the case of an assay for determining glycated and non-glycated hemoglobin the reagent chambers 108, 109,110 contained respectively:
a) a buffer and an amino phenyl boronate agarose (aPBA) matrix;
b) a wash buffer; and
c) an eluting buffer.

Each chamber pair can be brought into liquid communication via a channel (not shown) disposed in hub 103 when carousel 102 is rotated about hub 103.

In use device 101 is mounted vertically in an apparatus comprising drive means. A sample, for example a blood sample, is loaded in the first reagent chamber 108 via aperture 111 with device 101 in a first configuration in which there is no fluid communication between reagent chamber 108 and its diametrically opposed optical chamber 107. Once aperture 111 has been sealed device 101 is rotated back and forth through 120° to ensure thorough mixing, and in this example to facilitate lysing of the red blood cells thereby liberating the hemoglobin. Device 101 is left for 60-90 seconds during which the glycated hemoglobin present in the sample binds to the aPBA affinity matrix.

Once mixed carousel 102 is-held in place such that the first reagent chamber 108 is disposed above the first optical chamber 107 and hub 103 is rotated such that reagent chamber 108 communicates with optical chamber 107 via a tube (not shown), which contains a frit, in hub 103.

The contents of reagent chamber 108 collect in optical chamber 107 which contains the non-glycated hemoglobin present in the original sample. The aPBA affinity matrix collects in the tube frit (not shown) in hub 103.

Hub 103 is then held in place and carousel 102 is rotated a further 60° such that second reagent chamber 109 is brought into liquid communication with optical chamber 105 in a third configuration of device 101. This presents a wash for use and release of non-specifically bound non-glycated hemoglobin from the aPBA affinity matrix bound on the tube frit (not shown) of hub 103.

On rotating carousel 102 by a further 60° the third reagent chamber 110 containing the eluting solution is presented, in a fourth configuration of device 101. The elution buffer removes the glycated hemoglobin from the aPBA affinity matrix and is collected in optical chamber 106 for spectrophotometric analysis.

Referring now to device 1 in accordance with the invention which, in this embodiment, is mounted in hub 103 of device 101, device 1 comprises a cap 2 which, in use of device 1 in co-operation with device 101 covers carousel 102. Device 1 comprises a fluid sample receiving zone 3 which is in fluid communication (not shown in figure 1) with a reservoir (also not shown in figure 1) by means of a capillary tube 4. Device 1 further comprises a lens aperture 5 for viewing the contents of a excess fluid sample detection zone (not shown in figure 1).

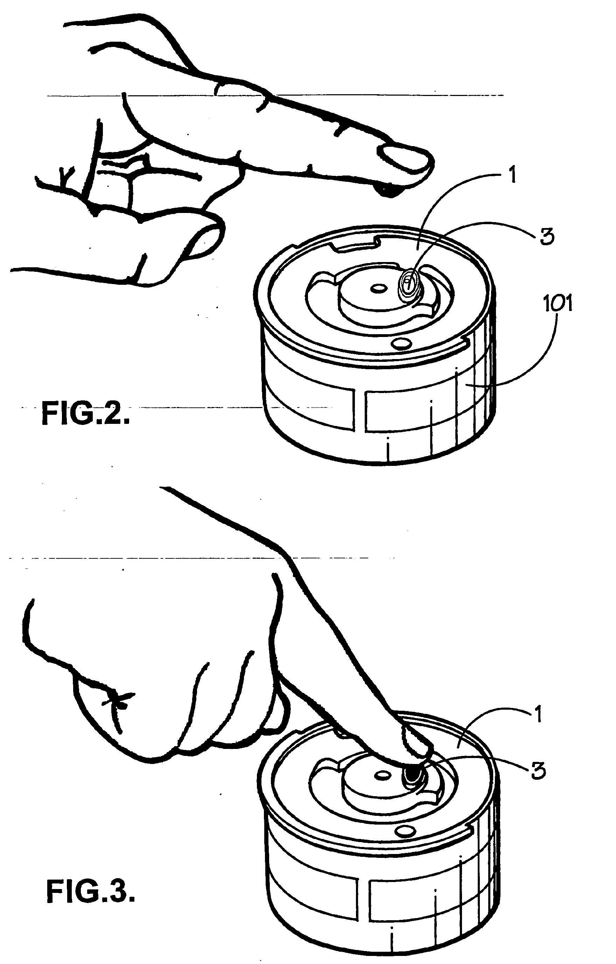

Figure 2 shows device 1 mounted on the hub of device 101 when device 101 is assembled. The assembled arrangement is shown prior to the addition of a blood sample to sample receiving zone 3.

Figure 3 shows a blood sample being supplied to sample receiving zone 3 of device 1.

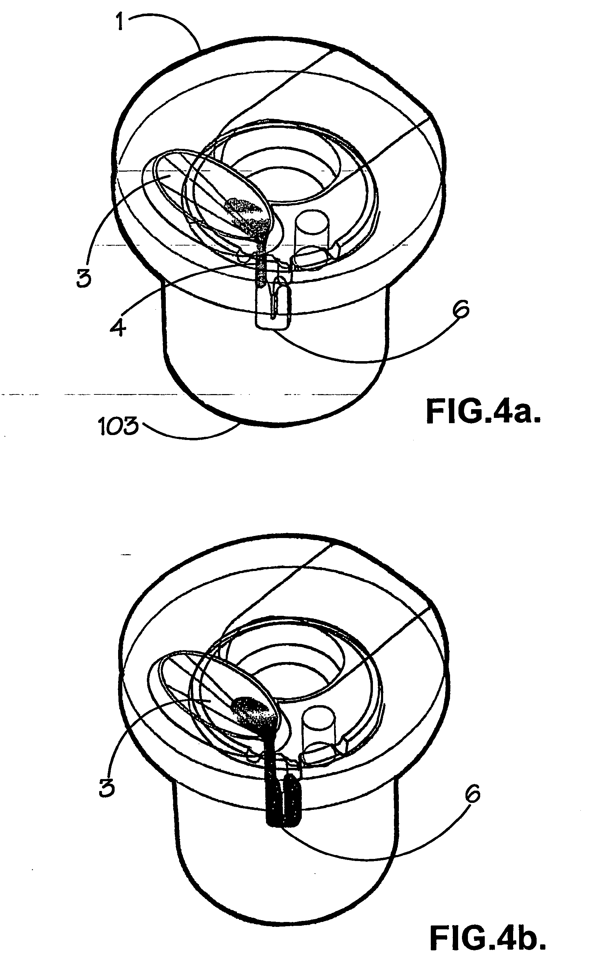

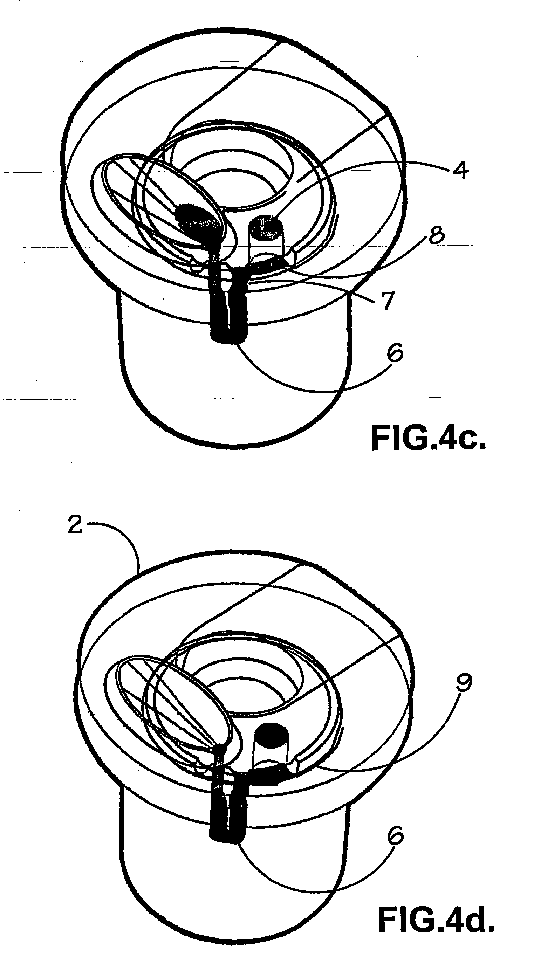

Figure 4a shows a partially cut away view of device 1 mounted on hub 103 of a device 101, all of which except for hub 103 is not shown in figure 4a, 4b, 4c of 4d. In figure 4a a blood fluid sample is shown in sample receiving zone 3 shortly after being supplied thereto. The blood sample is drawn by capillary action through tube 4 into reservoir 6.

Figure 4b shows the device of figure 4a a short while later, at which point reservoir 6 is completely full. However, the operator cannot yet see this in the device of figure 4b because reservoir 6 is, in use of the device, hidden from view.

Figure 4c shows the device of figure 4b a short while later. Overflow from reservoir 6 has been drawn by capillary action through tube 7 into an excess sample detection zone 8 viewable through lens aperture 4. At this stage, the operator is able to detect the presence of excess blood sample through lens aperture 4 and knows that reservoir 6 is completely full.

In figure 4d any further excess blood sample drawn by capillary action from the overflow of reservoir 6 is contained in an overflow well 9.

Once the operator has detected excess fluid sample in zone 8 and thereby knows that reservoir 6 is full, cap 2 can then be rotated to break the fluid communication between reservoir 6 and capillary tubes 4 and 7. The accurately determined quantity of fluid sample remaining in reservoir 6 can then be supplied to an assay component separation device, for example 101 of figure 1 to aperture 111 thereof. An assay procedure can then take place.

## Claims

1. A device for use in fluid assay comprising a sample reservoir for a fluid sample to be assayed, the reservoir (6) having an inlet port and an outlet port, the device comprising a fluid sample receiving zone (3) in fluid communication with the reservoir via the inlet port and an excess fluid sample detection zone (8) in fluid communication with the reservoir via the outlet port wherein the device has a first configuration in which fluid communication between the reservoir and each of the fluid sample receiving zone and the excess fluid sample detection zone is maintained and a second configuration in which one or each of said fluid communications is broken.

2. A device according to claim 1 wherein fluid communication between the fluid sample receiving zone and the reservoir is at least partly achieved by capillary action.

3. A device according to claim 1 or claim 2 wherein fluid communication between the fluid sample detection zone and the reservoir is at least partly achieved by capillary action.

4. A device according to any one of claims 1 to 3 wherein the reservoir has an overflow region and the excess fluid sample detection zone is in fluid communication with the overflow region of the reservoir.

5. A device as claimed in any one of claims 1 to 4 wherein rotational movement between the first and second configurations breaks the fluid communication between the reservoir and each of the fluid sample receiving zone and the excess fluid sample detection zone.

6. A device according to any one of claims 1 to 5 wherein the excess fluid sample detection zone is viewable through a lens aperture.

7. A device according to any one of claims 1 to 6 wherein the reservoir is sized to contain a pre-selected quantity of the sample to be assayed.

8. A device according to any one of claims 1 to 7 which is co-operable with drives means for use in automated fluid assay.

9. A device according to any one of claims 1 to 8 for use in assaying body fluid samples such as blood and urine.

10. A device according to any one of claims 1 to 9 which is co-operable with means for assaying the fluid sample.

11. An apparatus for fluid assay comprising a device in accordance with any one of claims 1 to 10 in co-operation with means for assaying the fluid sample.

12. A method for fluid assay comprising the steps of:
a) supplying a fluid to be assayed to the fluid sample receiving zone of a device according to any one of claims 1 to 10;
b) detecting excess fluid sample to be assayed in the excess fluid sample detection zone of the device;
c) optionally supplying the fluid sample in the reservoir to a device adapted for assaying the sample; and
d) assaying the sample.

## Patentansprüche

1. Vorrichtung zur Verwendung bei der Flüssigkeitsuntersuchung, umfassend einen Probebehälter für eine zu untersuchende Flüssigkeitsprobe, wobei der Behälter (6) einen Einlasskanal und einen Auslasskanal aufweist, wobei die Vorrichtung eine Flüssigkeitsproben-Aufnahmezone (3), die über den Einlasskanal mit dem Behälter in Flüssigkeitsverbindung steht und eine Flüssigkeitsprobenüberschuss-Erkennungszone (8), die über den Auslasskanal mit dem Behälter in Flüssigkeitsverbindung steht, umfasst, wobei die Vorrichtung eine erste Konfiguration hat, in der die jeweilige Flüssigkeitsverbindung zwischen dem Behälter und der Flüssigkeitsproben-Aufnahmezone und der Flüssigkeitsprobenüberschuss-Erkennungszone aufrecht erhalten wird und eine zweite Konfiguration, in der eine oder jede der Flüssigkeitsverbindungen unterbrochen ist.

2. Vorrichtung nach Anspruch 1, wobei die Flüssigkeitsverbindung zwischen der Flüssigkeitsproben-Aufnahmezone und dem Behälter mindestens teilweise durch Kapillarwirkung erreicht wird.

3. Vorrichtung nach Anspruch 1 oder Anspruch 2, wobei die Flüssigkeitsverbindung zwischen der Flüssigkeitsproben-Erkennungszone und dem Behälter mindestens teilweise durch Kapillarwirkung erreicht wird.

4. Vorrichtung nach einem der Ansprüche 1 bis 3, wobei der Behälter eine Überlaufregion hat und die Flüssigkeitsprobenüberschuss-Erkennungszone in Flüssigkeitsverbindung mit der Überlaufregion des Behälters steht.

5. Vorrichtung nach einem der Ansprüche 1 bis 4, wobei Drehbewegung zwischen der ersten und der zweiten Konfiguration die jeweilige Flüssigkeitsverbindung zwischen dem Behälter und der Flüssigkeitsproben-Aufnahmezone und der Flüssigkeitsprobenüberschuss-Erkennungszone unterbricht.

6. Vorrichtung nach einem der Ansprüche 1 bis 5, wobei die Flüssigkeitsprobenüberschuss-Erkennungzone durch ein Schauglas beobachtbar ist.

7. Vorrichtung nach einem der Ansprüche 1 bis 6, wobei die Größe des Behälters so bemessen ist, dass er eine vorherbestimmte Menge der zu untersuchenden Probe aufnehmen kann.

8. Vorrichtung nach einem der Ansprüche 1 bis 7, die für die Verwendung bei der automatischen Flüssigkeitsuntersuchung mit Antriebsmitteln zusammen wirksam ist.

9. Vorrichtung nach einem der Ansprüche 1 bis 8 für die Verwendung bei der Untersuchung von Körperflüssigkeitsproben, wie beispielsweise Blut oder Urin.

10. Vorrichtung nach einem der Ansprüche 1 bis 9, die mit Mitteln zur Untersuchung der Flüssigkeitsprobe zusammen wirksam ist.

11. Vorrichtung zur Flüssigkeitsuntersuchung, umfassend eine Vorrichtung nach einem der Ansprüche 1 bis 10 in Zusammenwirkung mit Mitteln zum Untersuchen der Flüssigkeitsprobe.

12. Verfahren zur Flüssigkeitsuntersuchung, umfassend folgende Schritte:
a) Zuführen einer zur untersuchenden Flüssigkeit an die Flüssigkeitsproben-Aufnahmezone einer Vorrichtung nach einem der Ansprüche 1 bis 10;
b) Erkennen von Überschuss der zu untersuchenden Flüssigkeitsprobe in der Flüssigkeitsprobenüberschuss-Erkennungszone der Vorrichtung;
c) optionales Zuführen der Flüssigkeitsprobe im Behälter an eine zum Untersuchen der Probe angepasste Vorrichtung; und
d) Untersuchen der Probe.

## Revendications

1. Dispositif destiné à être utilisé dans l'analyse de fluides qui comprend un réservoir à échantillon pour un échantillon fluide à analyser, le réservoir (6) comportant un orifice d'entrée et un orifice de sortie, le dispositif comprenant une zone de réception d'échantillon fluide (3) en communication fluidique avec le réservoir par l'intermédiaire de l'orifice d'entrée et une zone de détection de l'excès d'échantillon fluide (8) en communication fluidique avec le réservoir par l'intermédiaire de l'orifice de sortie où le dispositif a une première configuration dans laquelle la communication fluidique entre le réservoir et chacune de la zone de réception d'échantillon fluide et de la zone de détection de l'excès d'échantillon fluide est maintenue et une deuxième configuration dans laquelle l'une ou chacune desdites communications fluidiques est interrompue.

2. Dispositif selon la revendication 1 dans lequel la communication fluidique entre la zone de réception d'échantillon fluide et le réservoir est au moins en partie obtenue par action capillaire.

3. Dispositif selon la revendication 1 ou 2 dans lequel la communication fluidique entre la zone de détection d'échantillon fluide et le réservoir est au moins en partie obtenue par action capillaire.

4. Dispositif selon l'une quelconque des revendications 1 à 3 dans lequel le réservoir comporte une zone de débordement et la zone de détection de l'excès d'échantillon fluide est en communication fluidique avec la zone de débordement du réservoir.

5. Dispositif selon l'une quelconque des revendications 1 à 4 dans lequel le mouvement de rotation entre les première et deuxième configurations interrompt la communication fluidique entre le réservoir et chacune de la zone de réception d'échantillon fluide et de la zone de détection de l'excès d'échantillon fluide.

6. Dispositif selon l'une quelconque des revendications 1 à 5 dans lequel la zone de détection de l'excès d'échantillon fluide est visualisable à travers une ouverture de lentille.

7. Dispositif selon l'une quelconque des revendications 1 à 6 dans lequel le réservoir est dimensionné pour contenir une quantité prédéfinie de l'échantillon à analyser.

8. Dispositif selon l'une quelconque des revendications 1 à 7 qui peut être utilisé conjointement à des moyens d'entraînement destinés à être utilisés dans une analyse de fluides automatisée.

9. Dispositif selon l'une quelconque des revendications 1 à 8 destiné à être utilisé pour analyser des échantillons fluides organiques tels que le sang et l'urine.

10. Dispositif selon l'une quelconque des revendications 1 à 9 qui peut être utilisé conjointement à des moyens pour analyser l'échantillon fluide.

11. Appareil d'analyse de fluides comprenant un dispositif selon l'une quelconque des revendications 1 à 10 fonctionnant conjointement avec des moyens pour analyser l'échantillon fluide.

12. Méthode d'analyse de fluides comprenant les étapes consistant à :
a) alimenter un fluide à analyser vers la zone de réception d'échantillon fluide d'un dispositif selon l'une quelconque des revendications 1 à 10 ;
b) détecter l'excès d'échantillon fluide à analyser dans la zone de détection de l'excès d'échantillon fluide du dispositif ;
c) alimenter éventuellement l'échantillon fluide dans le réservoir vers un dispositif adapté pour analyser l'échantillon ; et
d) analyser l'échantillon.
